(19) Europäisches Patentamt
European Patent Office
Office européen des brevets

(11) EP 2 150 533 B1

(12) **EUROPEAN PATENT SPECIFICATION**

(45) Date of publication and mention of the grant of the patent:
**14.07.2010 Bulletin 2010/28**

(21) Application number: **08749150.2**

(22) Date of filing: **25.04.2008**

(51) Int Cl.:
***C07D 215/18*** *(2006.01)*

(86) International application number:
**PCT/EP2008/003374**

(87) International publication number:
**WO 2008/131932 (06.11.2008 Gazette 2008/45)**

(54) **PROCESS FOR THE PREPARATION OF OPTICALLY ACTIVE ETHENYLPHENYL ALCOHOLS**

VERFAHREN ZUR HERSTELLUNG OPTISCH AKTIVER ETHENYLPHENYLALKOHOLE

PROCÉDÉ DE PRÉPARATION D'ALCOOLS ÉTHÉNYLPHÉNYLIQUES OPTIQUEMENT ACTIFS

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**RS**

(30) Priority: **25.04.2007 EP 07008412**

(43) Date of publication of application:
**10.02.2010 Bulletin 2010/06**

(73) Proprietor: **Lonza AG**
**4052 Basel (CH)**

(72) Inventors:
- **MCGARRITY, John**
  **3902 Brig-Glis (CH)**
- **BAPPERT, Erhard**
  **4056 Basel (CH)**
- **BELSER, Thomas**
  **3900 Brig (CH)**

(56) References cited:
**EP-A- 0 480 717 US-A1- 2006 223 999**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

The present invention refers to a process for the preparation of optically active alcohols of formula

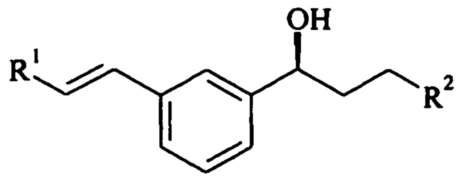

(I)

or its mirror image, wherein $R^1$ is unsubstituted or substituted heteroaryl and $R^2$ is phenyl or substituted aryl, by asymmetrically hydrogenating the corresponding ketones in the presence of specific platinum metal complex catalysts, particularly ruthenium.

Formula I comprises intermediates for the preparation of leukotriene antagonists being useful anti-asthmatic, anti-allergic, anti-inflammatory and cytoprotective therapeutic agents. Leukotriene antagonists are chiral compounds. One of their most prominent representatives is montelukast, the physiological active form of which is the *(R)* stereoisomer. The formation of the chiral center of formula I is a key step along the route to the final leukotriene antagonist as the hydrogenation of the corresponding ketones must be performed in a way that the alcohols obtained are optically enriched or pure. As these ketones additionally possess an olefinic bond, hydrogenation with standard hydrogenation catalysts followed by optical resolution is not the method of choice. The reason is that the olefinic bond is prone to be hydrogenated simultaneously with or even before the keto group. Therefore it is a big challenge to perform the hydrogenation both in a stereoselective and chemoselective manner.

It is known that the compounds of formula I can be obtained by reduction of the corresponding ketones with stoichiometric amounts of chiral reducing agents, particularly borane derivatives. For example EP 0 480 717 and US 2006/0223999 disclose use of oxazaborolidine complexes and EP 0 480 717 additionally describes application of *B*-chlorodiisopinocampheylborane ("DIP chloride"). If borane derivatives are the reducing agents, specialized and expensive facilities for the handling of these awkward and costly reagents are needed, especially for commercial production. Catalytic transfer hydrogenation is another approach for obtaining substances of formula I as disclosed in WO 2006/008562. However, this kind of reaction results in moderate yields and low robustness of the reaction, while relatively large amounts of expensive catalyst are required.

Consequently, there is a high need for a catalytic asymmetric hydrogenation by which cheap hydrogen gas can be applied in standard, large-scale production units.

The object described above is achieved by the process of claim 1.

After numerous, unsuccessful experiments with different catalysts all providing the undesired ethenyl hydrogenation (see comparison examples), applicants have surprisingly found that it is possible to prepare optically active alcohols of formula

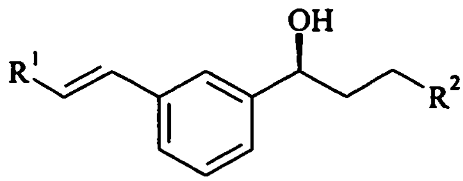

(I)

or its mirror image, wherein $R^1$ is unsubstituted or substituted heteroaryl and $R^2$ is phenyl or substituted aryl,
by asymmetric hydrogenation of a ketone of formula

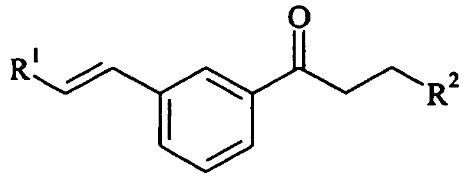

(II)

wherein $R^1$ and $R^2$ are as defined above,
with hydrogen gas in the presence of a platinum metal complex catalyst comprising a chiral phosphine ligand, wherein the platinum metal is selected from the group consisting of ruthenium, rhodium and iridium; and the chiral phosphine ligand is of formula

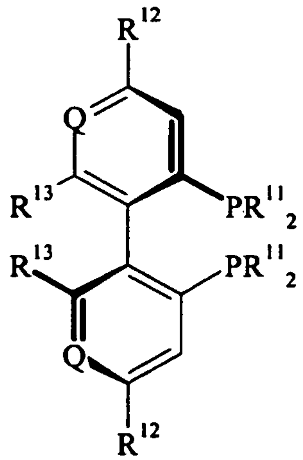

(III)

or its mirror image,
wherein
each $R^{11}$ is phenyl, 4-methylphenyl, 3,5-dimethylphenyl, furanyl or cyclohexyl;
each $R^{12}$ is hydrogen, $C_{1-4}$ alkyl or $C_{1-4}$ alkoxy;
and wherein

(a) each Q is nitrogen,
and each $R^{13}$ is $C_{1-4}$ alkyl or $C_{1-4}$ alkoxy; or
(b) each Q is $=CR^{14}-$, $R^{14}$ being selected from the group consisting of hydrogen, fluorine, chlorine, bromine, iodine and $C_{1-4}$ alkoxy,
and each $R^{13}$ is $C_{1-4}$ alkyl or $C_{1-4}$ alkoxy; or
(c) each Q is =CH-,
and both $R^{13}$ groups together form a moiety of formula $-O-(CH_2)_n-O-$, wherein n is an integer from 1 to 6; or
(d) each Q is $=CR^{15}-$, and each $R^{15}$ together with $R^{13}$ bound to the same benzene ring and together with said benzene ring form a ring system selected from the group consisting of naphthalene, tetralin, 2,3-dihydro-benzo[1,4]dioxine, unsubstituted or substituted benzo[1,3]dioxole, and N-methyl-2,3-dihydro-benzo[1,4]oxazine.

Here and as follows, the term "platinum metal" means the group VIII transition metals ruthenium, rhodium, palladium, osmium, iridium and platinum.

Here and as follows, the term "$C_{1-n}$ alkyl" is to be understood to mean any linear or branched alkyl group containing 1 to n carbon atoms. For example, the term "$C_{1-6}$ alkyl" comprises groups such as methyl, ethyl, propyl, isopropyl, butyl, isobutyl, *sec*-butyl, *tert*-butyl, pentyl, isopentyl (3-methylbutyl), neopentyl (2,2-dimethylpropyl), hexyl, isohexyl (4-methylpentyl) and the like.

Accordingly, the term "$C_{1-n}$, alkoxy" means a group composed of a $C_{1-n}$ alkyl group as defined above and an oxygen atom linked by a single covalent bond.

Here and as follows, the term "substituted benzo[1,3]dioxole" means a benzo[1,3]dioxole ring which is disubstituted at position 2 with two halogen, two $C_{1-4}$ alkyl or two $C_{1-4}$ alkoxy, respectively.

Here and as follows, the term "halogen" means fluorine, chlorine, bromine, iodine.

Examples of chiral phosphine ligands of formula III, in (*R*) or (*S*) configuration, are: "P-Phos", wherein Q is nitrogen, $R^{11}$ is phenyl and $R^{12}$ and $R^{13}$ are methoxy, i.e. 2,2',6,6'-tetramethyoxy-4,4'-bis(diphenylphosphino)-3,3'-bipyridine;

"Xyl-P-Phos", wherein Q is nitrogen, $R^{11}$ is 3,5-dimethylphenyl and $R^{12}$ and $R^{13}$ are methoxy, i.e. 2,2',6,6'-tetramethoxy-4,4'-bis[di(3,5-dimethylphenyl)phosphino]-3,3'-bipyridine;

"Tol-P-Phos", wherein Q is nitrogen, $R^{11}$ is 4-methylphenyl and $R^{12}$ and $R^{13}$ are methoxy, i.e. 2,2',6,6'-tetramethoxy-4,4'-bis[di(4-methylphenyl)phosphino]-3,3'-bipyridine;

"MeO-Biphep", wherein Q is =$CR^{14}$-, $R^{11}$ is phenyl, $R^{12}$ is hydrogen, $R^{13}$ is methoxy and $R^{14}$ is hydrogen, i.e. 6,6'-dimethoxy-2,2'-bis(diphenylphosphino)-1,1'-biphenyl;

"3',5'-Me2-MeO-Biphep", wherein Q is =$CR^{14}$-, $R^{11}$ is 3,5-dimethylphenyl, $R^{12}$ is hydrogen, $R^{13}$ is methoxy and $R^{14}$ is hydrogen, i.e. 6,6'-dimethoxy-2,2'-bis[di(3,5-dimethylphenyl)-phosphino]-1,1'-biphenyl;

"BIMOP", wherein Q is =$CR^{14}$-, $R^{11}$ is phenyl, $R^{12}$ and $R^{13}$ are methyl and $R^{14}$ is methoxy, i.e. 5,5'-dimethoxy-4,4',6,6'-tetramethyl-2,2'-bis(diphenylphosphino)-1,1'-biphenyl;

The compound, wherein Q is =$CR^{14}$-, $R^{11}$ is 2-furanyl, $R^{12}$ is hydrogen, $R^{13}$ is methoxy and $R^{14}$ is hydrogen, i.e. 6,6'-dimethoxy-2,2'-bis[di(2-furanyl)phosphino]-1,1'-biphenyl;

"Cl-MeO-Biphep", wherein Q is =$CR^{14}$-, $R^{11}$ is phenyl, $R^{12}$ is hydrogen, $R^{13}$ is methoxy and $R^{14}$ is chlorine, i.e. 6,6'-dimethoxy-5,5'-dichloro-2,2'-bis(diphenylphosphino)-1,1'-biphenyl;

"Bichep", wherein Q is =$CR^{14}$-, $R^{11}$ is cyclohexyl, $R^{12}$ is hydrogen, $R^{13}$ is methyl and $R^{14}$ is hydrogen, i.e. 6,6'-dimethyl-2,2'-bis(dicyclohexylphosphino)-1,1'-biphenyl;

"Biphemp", wherein Q is =$CR^{14}$-, $R^{11}$ is phenyl, $R^{12}$ is hydrogen, $R^{13}$ is methyl and $R^{14}$ is hydrogen, i.e. 6,6'-dimethyl-2,2'-bis(diphenylphosphino)-1,1'-biphenyl;

"C1-TunePhos", wherein Q is =CH-, $R^{11}$ is phenyl, $R^{12}$ is hydrogen, and both $R^{13}$ together are $-O-CH_2-O-$, i.e. 6,6'-bis (diphenylphosphino)-2,2'-methylenedioxybiphenyl;

"C2-TunePhos", wherein Q is =CH-, $R^{11}$ is phenyl, $R^{12}$ is hydrogen, and both $R^{13}$ together are $-O-(CH_2)_2-O-$, i.e. 1, 12-bis(diphenylphosphino)-6,7-dihydro-dibenzo[*e.g*][1,4]dioxocine;

"C3-TunePhos", wherein Q is =CH-, $R^{11}$ is phenyl, $R^{12}$ is hydrogen, and both $R^{13}$ together are $-O-(CH_2)_3-O-$, i.e. 1,13-bis(diphenylphosphino)-7,8-dihydro-6*H*-dibenzo[*f,h*][1,5]dioxonine;

"C4-TunePhos", wherein Q is =CH-, $R^{11}$ is phenyl, $R^{12}$ is hydrogen, and both $R^{13}$ together are $-O-(CH_2)_4-O-$, i.e. 1,14-bis(diphenylphosphino)-6,7,8,9-tetrahydrodibenzo[*g,i*][1,6]dioxecine;

"C5-TunePhos", wherein Q is =CH-, $R^{11}$ is phenyl, $R^{12}$ is hydrogen, and both $R^{13}$ together are $-O-(CH_2)_5-O-$, i.e., 1,15-bis(diphenylphosphino)-8,14-dioxa-tricyclo[13.4.0.0$^{2,7}$]nonadeca-1(15),2(7),3,5,16,18-hexaene;

"C6-TunePhos", wherein Q is =CH-, $R^{11}$ is phenyl, $R^{12}$ is hydrogen, and both $R^{13}$ together are $-O-(CH_2)_6-O-$, i.e. 1,16-bis(diphenylphosphino)-6,7,8,9,10,11-hexahydro-5,12-dioxa-di-benzo[a,c]cyclododecene;

"Binap", wherein Q is =$CR^{15}$-, $R^{11}$ is phenyl, $R^{12}$ is hydrogen, and each $R^{15}$ together with $R^{13}$ bound to the same benzene ring and together with said benzene ring form a naphthalene ring system, i.e. 2,2'-bis(diphenylphosphino)-1,1'-binaphthalene;

"TolBinap", wherein Q is =$CR^{15}$-, $R^{11}$ is 4-methylphenyl, $R^{12}$ is hydrogen, and each $R^{15}$ together with $R^{13}$ bound to the same benzene ring and together with said benzene ring form a naphthalene ring system, i.e. 2,2'-bis[di(4-methylphenyl) phosphino]-1,1'-binaphthalene;

"XylBinap", wherein Q is =$CR^{15}$-, $R^{11}$ is 3,5-dimethylphenyl, $R^{12}$ is hydrogen, and each $R^{15}$ together with $R^{13}$ bound to the same benzene ring and together with said benzene ring form a naphthalene ring system, i.e. 2,2'-bis[di(3,5-dimethylphenyl)phosphino]-1,1'-binaphthalene; "$H_8$-Binap", wherein Q is =$CR^{15}$-, $R^{11}$ is phenyl, $R^{12}$ is hydrogen, and each $R^{15}$ together with $R^{13}$ bound to the same benzene ring and together with said benzene ring form a tetralin ring system, i.e. 6,6'-bis(diphenylphosphino)- 5,5'-bitetralin;

"Synphos", wherein Q is =$CR^{15}$-, $R^{11}$ is phenyl, $R^{12}$ is hydrogen, and each $R^{15}$ together with $R^{13}$ bound to the same benzene ring and together with said benzene ring form a 2,3-dihydrobenzo[1,4]dioxine ring system, i.e. 2,2',3,3'-tetrahydro-6,6'-bis(diphenylphosphino)- 5,5'-bi(benzo[1,4]dioxin);

"Segphos", wherein Q is =$CR^{15}$ -, $R^{11}$ is phenyl, $R^{12}$ is hydrogen, and each $R^{15}$ together with $R^{13}$ bound to the same benzene ring and together with said benzene ring form a benzo[1,3]dioxole ring system, i.e. 5,5'-bis(diphenylphosphino)-4,4'-bi(benzo[1,3]dioxole);

"Difluorphos", wherein Q is =$CR^{15}$-, $R^{11}$ is phenyl, $R^{12}$ is hydrogen, and each $R^{15}$ together with $R^{13}$ bound to the same benzene ring and together with said benzene ring form a 2,2-difluorobenzo[1,3]dioxole ring system, i.e. 2,2,2',2'-tetrafluoro-5,5'-bis(diphenylphosphino)-4,4'-bi-(benzo[1,3]dioxole);

The compound, wherein Q is =$CR^{15}$-, $R^{11}$ is phenyl, $R^{12}$ is hydrogen, and each $R^{15}$ together with $R^{13}$ bound to the same benzene ring and together with said benzene ring form a 2,2-dimethylbenzo[1,3]dioxole ring system, i.e. 2,2,2',2'-tetramethyl-5,5'-bis(diphenylphosphino)-4,4'-bi(benzo[1,3]dioxole);

"Solphos", wherein Q is $=CR^{15}-$, $R^{11}$ is phenyl, $R^{12}$ is hydrogen, and each $R^{15}$ together with $R^{13}$ bound to the same benzene ring and together with said benzene ring form a *N*-methyl-2,3-dihydro-benzo[1,4]oxazine, i.e. *N,N*-dimethyl-2,2',3,3'-tetrahydro-7,7'-bis(diphenylphosphino) 8,8'-bi(benzo[1,4]oxazine) and

"XylSolphos", wherein Q is $=CR^{15}-$, $R^{11}$ is 3,5-dimethylphenyl, $R^{12}$ is hydrogen, and each $R^{15}$ together with $R^{13}$ bound to the same benzene ring and together with said benzene ring form a *N*-methyl-2,3-dihydro-benzo[1,4]oxazine, i.e. *N, N'*-dimethyl-2,2",3,3'-tetrahydro-7,7'-bis[di(3,5-dimethylphenyl)phosphino]-8,8'-bi(benzo[1,4]oxazine).

Particularly preferred are phosphines of formula III wherein Q is $=CR^{15}-$, $R^{12}$ is hydrogen, and each $R^{15}$ together with $R^{13}$ bound to the same benzene ring and together with said benzene ring form a naphthalene ring system. The use of "Binap" has proved particularly advantageous.

In another preferred embodiment the herein disclosed platinum metal complex catalyst further comprises a chiral diamine ligand of formula

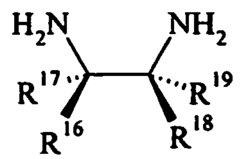

(IV)

wherein $R^{16}$ through $R^{19}$ are each independently hydrogen, cycloalkyl, linear or branched $C_{1-6}$ alkyl, or phenyl optionally substituted with one or more $C_{1-4}$ alkyl or $C_{1-4}$ alkoxy groups, as these ligands showed a favourable effect on the chemoselectivity of the reaction.

The term "cycloalkyl" is to be understood to mean mono- or bicyclic saturated groups such as cyclopropyl, cyclobutyl, cyclopentyl, cyclohexyl, norbornyl, norcaryl, norpinanyl, and related groups, such as the above-mentioned groups being further substituted with lower alkyl substituents.

Examples of such chiral diamine ligands of formula IV are, particularly in *(R)* and *(S)* configuration for DAIPEN and in (*R,R*) and (*S,S*) configuration for DPEN:

"DAIPEN", wherein $R^{16}$ is isopropyl; $R^{17}$ is hydrogen; and $R^{18}$ and $R^{19}$ are 4-methoxyphenyl, i.e. 1,1-bis(4-methoxyphenyl)-3-methyl-1,2-butanediamine and

"DPEN", wherein $R^{16}$ and $R^{19}$ are phenyl; and $R^{17}$ and $R^{18}$ are hydrogen, i.e. 1,2-diphenylethylenediamine.

In a preferred embodiment the chiral diamine ligand of formula IV is "DAIPEN".

In a particular embodiment, $R^1$ is a heterocyclic group of formula

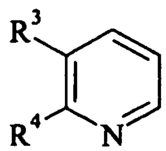

(V)

wherein $R^3$ and $R^4$ together form a moiety of formula $-S-CR^5=CR^6-$, with the proviso that the sulfur atom is directly bound to the carbon atom in position 3 of the pyridine moiety;

or alternatively $R^3$ and $R^4$ together form a moiety of formula $-CR^5=CR^6-CR^7=CR^8-$, with the proviso that the carbon atom attached to $R^5$ is directly bound to the carbon atom in position 3 of the pyridine moiety;

and each of $R^5$ through $R^8$ is independently hydrogen or halogen.

Also particularly, $R^2$ is $-C_6H_4R^9$, wherein $R^9$ is selected from the group consisting of halogen, $C_{1-4}$ alkyl, branched and linear $C_{2-4}$ alkenyl, $C_{5-6}$ cycloalkyl, phenyl, $C_{1-4}$ alkoxy, $C_{1-4}$ alkylthio, carboxy, ($C_{1-4}$ alkoxy)carbonyl, ($C_{1-4}$ alkoxy)sulfonyl, $-T-O-R^{10}$, wherein T is branched or linear $C_{1-8}$ alkanediyl and $R^{10}$ is selected from the group consisting of hydrogen, methyl, substituted methyl, substituted ethyl, phenyl, substituted phenyl, substituted benzyl, pyridinylmethyl, substituted pyridinylmethyl, substituted silyl, $C_{1-6}$ acyl, substituted $C_{1-6}$ acyl, ($C_{1-4}$ alkoxy)carbonyl, substituted ($C_{1-4}$ alkoxy)carbonyl and aryloxycarbonyl; and $R^9$ may be located at any position of the phenyl ring.

Here and as follows, the term "$C_{2-n}$ alkenyl" is to be understood to mean a carbon chain containing at least one double bond located at any position of the carbon chain. For example, the term "$C_{2-4}$ alkenyl" comprises groups such as ethenyl, 1-methylethenyl, prop-1-enyl, prop-2-enyl, 2-methylprop-2-enyl and buta-1,3-dienyl.

Here and as follows, the term "$C_{1-n}$ alkylthio" means a group composed of a $C_{1-n}$ alkyl group as defined above

and an sulfur atom linked by a single covalent bond.

Here and as follows, the term "($C_{1-n}$ alkoxy)carbonyl" means a carboxylic acid ester derived from a $C_{1-n}$ alkanol which might be linear or branched. For example, the term "($C_{1-4}$ alkoxy)-carbonyl" comprises groups such as methoxycarbonyl, ethoxycarbonyl, propoxycarbonyl, isopropoxycarbonyl, butoxycarbonyl, 2-methylpropoxy and *tert*-butoxycarbonyl.

Here and as follows, the term "($C_{1-n}$ alkoxy)sulfonyl" is analogous to the term "($C_{1-n}$ alkoxy)-carbonyl" as described above except for sulfonic acid ester instead of carboxylic acid ester.

Here and as follows, the term "substituted methyl" for $R^{10}$ means a single carbon atom directly linked to the oxygen of the -T-O-$R^{10}$ moiety while also linked at least to one heteroatom or carbon atom optionally forming a cyclic ring system thereby. Examples for "substituted methyl" are methoxymethyl, ethoxymethyl, methylthiomethyl, tert-butylthiomethyl, (phenyldimethylsilyl)methyoxymethyl, benzyloxymethyl, 4-methoxybenzyloxymethyl, (4-methoxyphenoxy)methyl, (2-methoxyphenoxy)methyl, tert-butoxymethyl, 4-pentenyloxymethyl, siloxymethyl, 2-methoxyethoxymethyl, 2,2,2-trichloroethoxymethyl, bis(2-chloroethoxy)methyl, 2-trimethylsilyl)ethoxymethyl, benzyl, diphenylmethyl, triphenylmethyl, tetrahydropyranyl, 3-bromotetrahydropyranyl, tetrahydrothiopyranyl, 1-methoxycyclohexyl, 4-methoxytetrahydropyranyl, 4-methoxytetrahydrothiopyranyl, S,S-dioxo-4-methoxytetrahydrothiopyranyl, 1-(2-chloro-4-methylphenyl)-4-methoxypiperidin-4-yl, 1,4-dioxan-2-yl, tetrahydrofuranyl, tetrahydrothiofuranyl and 2,3,3a,4,5,6,7,7a-octahydro-7,8,8-trimethyl-4,7-methanobenzofuran-2-yl.

Here and as follows, the term "substituted ethyl" for $R^{10}$ means an ethyl group which in position 1 is directly linked to the oxygen of the -T-O-$R^{10}$ moiety while said ethyl group is substituted in position 1 and/or position 2 by at least one substituent wherein the substituent is linked to the ethyl group by carbon-carbon, carbon-heteroatom, carbon-silicon and/or carbon-selenium bonding. Examples for "substituted ethyl" are 1-ethoxyethyl, 1-(2-chloroethoxy)ethyl, 1-methyl-1-methoxyethyl, 1-methyl-1-benzyloxyethyl, 1-methyl-1-benzyloxy-2-fluoroethyl, 2,2,2-trichloroethyl, 2-trimethylsilylethyl, 2-(phenylselenyl)ethyl, *tert*-butyl and allyl.

Here and as follows, the term "substituted phenyl" for $R^{10}$ means a phenyl group directly linked to the oxygen of the -T-O-$R^{10}$ moiety, wherein the phenyl is substituted at least with halogen, nitro, $C_{1-4}$ alkyl or $C_{1-4}$ alkoxy. Examples for "substituted phenyl" are 4-chlorophenyl, 4-methoxyphenyl and 2,4-dinitrophenyl.

Here and as follows, the term "substituted benzyl" for $R^{10}$ means a carbon atom which is directly linked to the oxygen of the -T-O-$R^{10}$ moiety and which is additionally linked to at least one substituted phenyl group. Examples for "substituted benzyl" are 4-methoxybenzyl, 3,4-dimethoxybenzyl, 2-nitrobenzyl, 4-nitrobenzyl, 4-halobenzyl, 2,6-dichlorobenzyl, 4-cyanobenzyl, 4-phenylbenzyl, 4,4'-dinitrobenzhydryl, 10,11-dihydro-5*H*-dibenzo[*a,d*]cycloheptene-5-yl, α-naphthyldiphenylmethyl, (4-methoxyphenyl)diphenylmethyl, di(4-methoxyphenyl)-phenylmethyl, tri(4-methoxyphenyl) methyl, [4-(4'-bromophenacyloxy)phenyl]diphenylmethyl, tris[4-(4,5-dichlorophthalimido)phenyl]methyl, tris[4-(4-oxopentanoyl)phenyl]methyl, tris(4-benzyloxyphenyl)methyl, [3-(imidazol-1-ylmethyl)phenyl]bis(4-methoxyphenyl)methyl, bis(4-methoxyphenyl)(1-pyrenyl)methyl, 9-phenylxanthene-9-yl and 9-phenyl-10-oxoanthracene-9-yl.

Here and as follows, the term "pyridinylmethyl" for $R^{10}$ in the -T-O-$R^{10}$ moiety comprises 2-pyridine-x-ylmethyl and 4-pyridine-x-ylmethyl.

Here and as follows, the term "substituted pyridinylmethyl" for $R^{10}$ means a carbon atom which is directly linked to the oxygen of the -T-O-$R^{10}$ moiety and which is additionally linked to at least one substituted pyridinyl group. An example for "substituted pyridinylmethyl" is *N*-oxy-3-methyl-2-pyridine-x-ylmethyl.

Here and as follows, the term "substituted silyl" for $R^{10}$ means a silyl group which is directly linked to the oxygen of the -T-O-$R^{10}$ moiety and which is substituted with substituents independently selected from the group consisting of linear or branched $C_{1-10}$ alkyl, $C_{1-3}$ alkoxy, benzyl and phenyl optionally substituted with one or more $C_{1-4}$ alkyl or $C_{1-4}$ alkoxy. Examples for "substituted silyl" are trimethylsilyl, triethylsilyl, triisopropylsilyl, dimethylisopropylsilyl, diethylisopropylsilyl, dimethylhexylsilyl, tert-butyldimethylsily, *tert*-butyldimethylsilyl, *tert*-butyldiphenylsilyl, tribenzylsilyl, tri-*p*-xylylsilyl, triphenylsilyl, diphenylmethylsilyl and *tert*--butyl-methoxy-phenylsilyl.

Here and as follows, the term "$C_{1-6}$ acyl" for $R^{10}$ means an acyl group derived from a saturated or unsaturated carboxylic acid of 1 to 6 carbon atoms. Examples for "$C_{1-6}$ acyl" are formyl, acetyl, propanoyl and but-2-enoyl.

Here and as follows, the term "substituted $C_{1-6}$ acyl" for $R^{10}$ means the acyl group as defined above which is substituted with at least one substituent independently selected from the group consisting of $C_{1-4}$ alkyl, halogen, oxo, $C_{1-4}$ alkoxy optionally substituted with phenyl, phenoxy, phenyl optionally substituted with one or more $C_{1-4}$ alkyl, phenyl or halogen, and 1-adamantyl. Examples for "substituted $C_{1-6}$ acyl" are 2-oxo-2-phenylacetyl, chloroacetyl, dichloroacetyl, trichloroacetyl, trifluoroacetyl, triphenylmethoxyacetyl, methoxyacetyl, phenoxyacetyl, 4-chlorophenylacetyl, 3-phenylpropanoyl, 4-oxopentanoyl, 2,2-dimethylpropanoyl, 1-adamantylformyl, 4-methoxybut-2-enoyl, benzoyl, 4-phenylbenzoyl and 2,4,6-trimethylbenzoyl.

Here and as follows, the term "substituted ($C_{1-4}$ alkoxy)carbonyl" for $R^{10}$ means a ($C_{1-4}$ alkoxy)carbonyl group as defined above, wherein the $C_{1-4}$ alkanol from which it is derived is substituted with at least one substituent independently selected from the group consisting of halogen, $C_{1-4}$ alkyl substituted silyl, benzenesulfonyl, vinyl, phenyl optionally substituted with one or more $C_{1-4}$ alkyl, $C_{1-4}$ alkoxy, halogen, nitro and fluorenyl. Examples for substituted "($C_{1-4}$ alkoxy)

carbonyl" are 9-fluorenylmethoxycarbonyl, 2,2,2-trichloroethoxycarbonyl, 2-trimethylsilylethoxycarbonyl, 2-benzenesulfonylethoxycarbonyl, prop-2-enyloxycarbonyl, benzyloxycarbonyl, (4-methoxyphenyl)methoxycarbonyl, (3,4-dimethoxyphenyl)methoxycarbonyl, (2-nitrophenyl)methoxycarbonyl and (4-nitrophenyl)methoxycarbonyl.

Here and as follows, the term "aryloxycarbonyl" for $R^{10}$ means a carboxylic acid ester derived from a $C_{6-18}$ phenol optionally substituted with one or more $C_{1-4}$ alkyl, $C_{1-4}$ alkoxy, halogen or nitro. An example for "aryloxycarbonyl" is 4-nitrophenoxycarbonyl.

In a preferred embodiment $R^1$ is of formula V, wherein $R^3$ and $R^4$ together form a moiety of formula $-CR^5{=}CR^6{-}CR^7{=}CR^8-$; and each of $R^5$ through $R^8$ is independently hydrogen or halogen; and $R^2$ is $-C_6H_4R^9$, wherein $R^9$ is ($C_{1-4}$ alkoxy)carbonyl or -T-O-$R^{10}$, wherein T is branched or linear $C_{1-8}$ alkanediyl and $R^{10}$ is hydrogen or substituted methyl; and $R^9$ is located at position 2 of the phenyl ring.

In a more preferred embodiment $R^1$ is of formula V, wherein $R^3$ and $R^4$ together form a moiety of $-CR^5{=}CR^6{-}CR^7{=}CR^8-$; $R^5$, $R^6$ and $R^8$ are hydrogen; and $R^7$ is chlorine; and wherein $R^2$ is $-C_6H_4R^9$, and $R^9$ is -T-O-$R^{10}$ wherein T is $-C(CH_3)_2-$ and $R^{10}$ is hydrogen or substituted methyl selected-from the group-consisting of tetrahydropyranyl, methoxymethyl and ethoxymethyl; and $R^9$ is located at position 2 of the phenyl ring.

In a most preferred embodiment $R^1$ is of formula V, wherein $R^3$ and $R^4$ together form a moiety of formula $-CR^5{=}CR^6{-}CR^7{=}CR^8-$, $R^5$, $R^6$ and $R^8$ are hydrogen and $R^7$ is chlorine; and wherein $R^2$ is $-C_6H_4R^9$, and $R^9$ is methoxycarbonyl and located at position 2 of the phenyl ring, i.e. methyl 2-[3-[(*E*)-3-(2-(7-chloro-2-quinolinyl)ethenyl)phenyl]-3-oxopropyl]benzoate.

The catalyst can be obtained by dissolving a suitable salt of the platinum metal selected from the group consisting of of ruthenium, rhodium or iridium, wherein suitable counterions are for example chloride, bromide, iodide, tetrafluoroborate, hexafluoroarsenate, hexafluoroantimonate, hexafluorophosphate, perchlorate or trifluoromethanesulfonate in a polar solvent with a suitable amount of the phosphine ligand followed by isolation of the complex formed.

In addition, these suitable salts of ruthenium, rhodium or iridium preferably comprise at least one stabilizing ligand, such as an alkene, alkanediene or arene. In a preferred embodiment the stabilizing ligand is 2-methylallyl, 1,5-cyclooctadiene, norbornadiene, phenyl or *p*-cymene. The thus stabilized metal salts may also comprise at least one polar molecule as additional stabilizing ligand coming from the solvent or an added base. Examples of such polar molecules are acetonitrile, dimethylsulfoxide, dimethylformamide and triethylamine.

Alternatively, the catalyst can be prepared *in situ* from the phosphine ligands and the salt of the platinum metal as defined above. Preferably, the catalyst is prepared *in situ* from the phosphine ligands and salts of ruthenium, rhodium or iridium which are either stabilized as described above or which are suitable precursor complexes such as $[RuCl_2(PPh_3)_3]$.

Optionally, the preparation of the catalyst can be performed in the presence of a chiral diamine ligand.

In a particularly preferred embodiment the salt is a ruthenium salt, the phosphine ligand is "Binap", and the chiral diamine is "DAIPEN". In a particularly preferred embodiment this ruthenium complex catalyst is [*(S)*-Binap $RuCl_2$ (*S*)-DAIPEN] and [(*R*)-Binap $RuCl_2$ (*R*)-DAIPEN].

A suitable preparation method of the "Binap" ligand is disclosed in D. Cai, J. F. Payack, D. R. Bender, D. L. Hughes, T. R. Verhoeven, P. J. Reider, Org. Synth. 1998, 76, 6-11. The isolated [Binap $RuCl_2$ DAIPEN] catalyst can be obtained by reacting $[RuCl_2(C_6H_6)]_2$, "Binap" and "DAIPEN" in dimethylformamide followed by re-crystallization from dichloromethane and diethyl ether (1:10).

The catalyst may be added to the reaction mixture as such or dissolved in a suitable solvent, or alternatively the catalyst may be prepared *in situ.*

The catalyst may also be polymer-bound by linkage of a suitable group of the phosphine ligand to a resin. Polymer-bound catalysts of this kind are particularly advantageous for simple purification of the product.

Bases applicable in the present invention include inorganic and organic bases. The bases may be expressed by the general formula MY, wherein M is an alkali metal or one equivalent of an alkaline earth metal, and Y is a hydroxy group, alkoxy group, carboxylate, hydrogencarbonate or one equivalent of carbonate. More specifically, applicable bases include NaOH, KOH, CsOH, $LiOCH_3$, $NaOCH_3$, $NaOCH(CH_3)_2$, $KOCH_3$, $KOCH(CH_3)_2$, $KOC(CH_3)_3$, $NaOCOCH_3$, $K_2CO_3$, $Na_2CO_3$, $Cs_2CO_3$, $CaCO_3$ and $BaCO_3$. Alternatively, the base may be an amine like *tert*-butylamine, dimethylamine, diethylamine, trimethylamine, triethylamine or triethylenediamine. Most preferably $K_2CO_3$, $Cs_2CO_3$ or NaOH is used as base.

As solvent, any inert liquid solvent which can dissolve the reactants and catalyst components may be used. Applicable solvents include aromatic hydrocarbons such as toluene and xylene; halogenated aromatic hydrocarbons such as chlorobenzene and trifluorotoluene; aliphatic hydrocarbons such as pentane and hexane; halogenated hydrocarbons such as dichloromethane and dichloroethene; ethers such as diethyl ether, tetrahydrofuran, 2-methyltetrahydrofuran; alcohols such as methanol, ethanol, 2-propanol, butanol and benzyl alcohol; halogenated alcohols such as 2,2,2-trifluoroethanol; carboxylic esters and lactones such as ethyl acetate, methyl acetate and valerolactone; and organic solvents containing heteroatoms such as acetonitrile, dimethylformamide and dimethyl sulfoxide. The solvents can be used alone; as mixture or two-phase system with water; or in mixtures of at least two solvents optionally in combination

with water. Preferred solvents are mixtures of halogenated aromatic hydrocarbons and alcohols.

Optionally, the reaction mixture may contain a Lewis acid such as scandium(III) triflate, bismuth(III) triflate, yttrium(III) triflate, copper(I) chloride, copper(II) chloride, magnesium chloride, aluminium chloride, iron(III) chloride, cerium(III) chloride, lanthanum(III) chloride, neodymium(III) chloride and samarium(III) chloride. If a Lewis acid appears as hydrate, this hydrate compound may be applied as well. Addition of Lewis acids may enhance both the enantioselectivity of the reaction and the stability of the catalyst.

Optionally, the reaction can be carried out in the presence of a phase transfer catalyst such as an ammonium halide. Examples of such ammonium halides are tetraethylammonium bromide, triethylbenzylammonium chloride (TEBA), tetrabutylammonium chloride, tetrabutylammonium bromide and tetrabutylammonium iodide. TEBA has been found to be particularly useful. Addition of phase transfer catalysts may have a positive effect on the separation of the product formed.

The amount of the ketone of formula II (substrate) varies with the reactor volume and can be at a molar ratio relative to the catalyst (S/C) from 100:1 to 100,000:1, or more preferably from 500:1 to 20,000:1.

The hydrogenation process according to the invention may be carried out at atmospheric pressure or superatmospheric pressure. Typical pressures are from 1 to 100 bar. Advantageously, 1 to 70 bar, in particular 5 to 40 bar are used. The chemoselectivity appears to be generally better with lower pressures.

The hydrogenation reactions may be carried out at low or elevated temperatures. An examplary temperature range is from -20 °C to 120 °C. Preferred is a temperature between 0 °C and 100 °C, and most preferred is a range from 10 °C to 40 °C.

The reaction time depends on different factors like the catalyst loading, the temperature and the hydrogen pressure. Therefore, the reaction may be completed in a period of time within a range from a few minutes to several hours or even days.

## Examples

The following examples further illustrate this invention but are not intended to limit it in any way.

The structure and stereochemistry of the comparison ligand 1 is as follows:

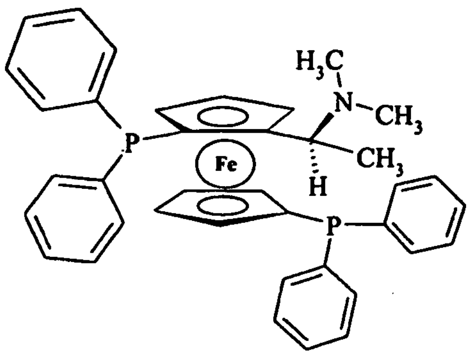

**1**

### Example 1: Synthesis of 1-[(*E*)-3-(2-(7-chloro-2-quinolinyl)ethenyl)phenyl]-2-propen-1-01

A suspension of (*E*)-3-(2-(7-chloro-2-quinolinyl)ethenyl)benzaldehyde (50 g, 0.17 mol, commercially available from Unibest Industrial Ltd., Ningbo, China) in 400 mL toluene was degassed at 0 °C. A 1.6 M solution of vinylmagnesium chloride in THF (115 mL, 0.18 mol) was added dropwise over 30 minutes while keeping the internal temperature at <10 °C. After stirring for 1 hour at 0-5 °C, the reaction mixture was quenched by slow addition of 400 mL aqueous ammonium acetate solution (10 %). The two-phase mixture thus obtained was stirred for 1 hour to ensure the solvolysis of the magnesium salts.

The separated organic layer was washed two times with 500 mL water and concentrated in vacuum to a volume of 75 mL. Then, again 75 mL acetonitrile were added and the mixture was concentrated in vacuum to 75 mL. After repetition of the last procedure, the resulting slurry was directly used in the next step.

### Example 2: Synthesis of methyl 2-[3-[(*E*)-3-(2-(7-chloro-2-quinolinyl)ethenyl)phenyl]-3-oxopropyl]benzoate

The crude slurry of example 1 in acetonitrile was treated with methyl 2-iodobenzoate (44.6 g, 0.17 mol), triethylamine (35.6 mL, 0.254 mol) and palladium acetate (0.36 g, 1.7 mmol). The mixture was degassed and heated at reflux under nitrogen for 6 hours. Then, the hot solution was filtered through cellulose (Solka Floc®) to remove any precipitated palladium. When the filtrate cooled to ambient temperature, the desired title product crystallized from solution. After one hour at ambient temperature, the suspension was filtered. The filter cake was washed consecutively with 130 mL acetonitrile, 100 mL acetonitrile/water (1:1), 100 mL water and finally 150 mL acetonitrile. After drying, the title product was obtained as a pale-yellow solid (53 g, 69% referring to the benzaldehyde of Example 1).
$^1$H NMR complies with EP 0 480 717 B (example 16, step 3).

**Examples 3 to 9 and comparison examples C1 to C3: Asymmetric hydrogenation of methyl 2-[3-[(*E*)-3-(2-(7-chloro-2-quinolinyl)ethenyl)phenyl]-3-oxopropyl]benzoate**

The examples 3 to 6 and C1 to C3 were performed by high throughput catalysis screening (HTS) using a HTS automated screening tool from Symyx Inc. and a customized Symyx Workflow. All reactions were performed in 1.2 mL vials on a 96-well plate placed in a high-pressure reactor (HIP). The whole HTS was performed in a glove box.

Procedure for examples 3 to 6 - exemplarily shown on example 3:

A stock solution of [(*S*)-Binap $RuCl_2$ (*S*)-DAIPEN] in dichloroethene (0.0017 mmol in 0.08 mL) was prepared and filled into a reaction vial. The solvent was completely removed under reduced pressure. A stock solution of the substrate methyl 2-[3-[(*E*)-3-(2-(7-chloro-2-quinolinyl)-ethenyl)phenyl]-3-oxopropyl]benzoate in tetrahydrofuran (0.042 mmol in 0.09 mL) was added. The solvent was completely removed under reduced pressure a second time. Stock solutions of potassium carbonate in water (0.046 mmol in 0.05 mL) and of triethylbenzylammonium chloride in 2-propanol (0.004 mmol in 0.06 mL) were added. Finally, the vial was filled with toluene until a total volume of 0.5 mL was reached. The reaction mixture was then purged four times with hydrogen, pressurised to 5 bar and run for 18 hours at room temperature.

General procedure for examples 7 to 9:

For example 8, the reaction mixture and hydrogenation conditions are identical to example 10, except for the reaction time. For examples 7 and 9, the reaction mixture was prepared analogous to example 10, but hydrogenation was performed in a 50 mL stainless steel autoclave and the product was isolated only by extraction.

Procedure for the comparison examples C1 to C3 - exemplarily shown on example C1:

A solution of the ligand 1 in dichloroethene (0.0020 mmol in 0.12 mL) was filled into a reaction vial. A stock solution of $[RuI_2(p\text{-cymene})]_2$ in dichloroethene (0.0008 mmol in 0.04 mL) was added, followed by toluene until a total volume of 0.40 mL was reached. The vial was closed, and the mixture was heated at 80°C for one hour. Then, the solvent was completely removed under reduced pressure and a stock solution of the substrate methyl 2-[3-[(*E*)-3-(2-(7-chloro-2-quinolinyl)ethenyl)phenyl]-3-oxopropyl]benzoate in tetrahydrofuran (0.042 mmol in 0.09 mL) was added. A stock solution of lithium methanolate in methanol (0.033 mmol in 0.10 mL) was also added. Finally, the vial was filled with 2-propanol until a total volume of 0.50 mL was reached. The reaction mixture was then purged four times with hydrogen, pressurized to 5 bar and run for 16 hours at room temperature.

Details of the examples 3 to 9 and C1 to C3 with regard to the catalyst used, reaction conditions and the results achieved are compiled in tables 1 and 2. Conversion and the product methyl 2-[3-[(*E*)-3-(2-(7-chloro-2-quinolinyl)ethenyl)phenyl]-3-hydroxypropyl]benzoate were calculated from uncorrected supercritical fluid chromatography (SFC) integrals. The enantiomeric excess (ee) was also measured by SFC (Chiralpak AD-H, 40% 2-propanol).

**Table 1: Examples 3 to 9** (room temperature, 5 bar of hydrogen)

| No | Cat | S/C | S [mmol] | Solvent mixture | Base/ Additive* | Time [h] | Conv [%] | Product [%] | ee [%] |
|---|---|---|---|---|---|---|---|---|---|
| 3 | (*S*) | 25 | 0.042 | IPA/toluene/$H_2O$ (1:6:1; 0.5 mL) | $K_2CO_3$/ TEBA | 18 | 100 | 98.3 | 95.4 (*R*) |
| 4 | (*S*) | 25 | 0.042 | Clbenz/THF/$H_2O$ (15:1:2; 0.5 mL) | $K_2CO_3$/ Y $(OTF)_3$ | 18 | 100 | 95.5 | 94.9 (*R*) |
| 5 | (*S*) | 25 | 0.042 | IPA, triFtoluene (1:4; 0.5 mL) | $Cs_2CO_3$/ - | 6 | 100 | 94.3 | 96.4 (*R*) |

(continued)

| No | Cat | S/C | S [mmol] | Solvent mixture | Base/ Additive* | Time [h] | Conv [%] | Product [%] | ee [%] |
|---|---|---|---|---|---|---|---|---|---|
| 6 | (*S*) | 100 | 0.083 | Clbenz/IPA/$H_2O$ (5:1:1.5; 0.5 mL) | NaOH/ TEBA | 6 | 99.4 | 97.6 | 95.7 (*R*) |
| 7 | (*R*) | 100 | 3.5 | Clbenz/IPA (5:1; 20 mL) | NaOH/ TEBA | 6 | 100 | > 99 | 96.9 (*S*) |
| 8 | (*R*) | 482 | 31.8 | Clbenz/IPA (5:1; 20 mL) | NaOH/ TEBA | 6 | > 95 | 99 | 96.7 (*S*) |
| 9 | (*R*) | 1000 | 3.5 | Clbenz/IPA (5:1; 20 mL) | NaOH TEBA | 30 | 99 | 99 | 96.6 (*S*) |

* = 1.1 equivalents base and 0.05-0.1 equivalents additive; cat = catalyst; *(S)* = [(*S*)-Binap $RuCl_2$ *(S)*-DAIPEN]; *(R)* = [*(R)*-Binap $RuCl_2$ *(R)*-DAIPEN]; (*S*)-Binap = (*S*)-(-)-2,2'-bis-(diphenylphosphino)-1,1'-binaphthalene; (S)-DAIPEN = (2*S*)-(-)-1,1-bis(4-methoxyphenyl)-3-methyl-1,2-butanediamine; IPA = 2-propanol; THF = tetrahydrofuran; Cl-benzene = chlorobenzene; triFtoluene = trifluorotoluene; $Y(OTF)_3$ = yttrium(III) triflate, TEBA = triethylbenzylammonium chloride.

**Table 2: Comparison examples C1 to C3** (IPA/THF/MeOH 3:1:1; 0.5 mL; room temperature; 5 bar of hydrogen)

| No | Pre | Lig | S/C | S [mmol] | Base/ Additive * | Time [h] | Conv [%] | Product [%] | By-Product (C=C hydrogenation) [%] |
|---|---|---|---|---|---|---|---|---|---|
| C1 | **a** | **1** | 25 | 0.042 | LiOMe/- | 16 | 82.6 | 0 | 54.1 |
| C2 | **b** | **1** | 25 | 0.042 | DABCO/- | 16 | 84.3 | 0 | 75.0 |
| C3 | **a** | **2** | 25 | 0.042 | LiOMe/- | 16 | 90.3 | 3.9 | 44.9 |

Pre = precursor catalyst; Lig = ligand; * = 1.1 equivalents base; **a** = $[RuI_2(p\text{-cymene})]_2$; **b** = $[Ir(1,5\text{-cyclooctadiene})_2]$ BARF with BARF = tetrakis[3,5-bis(trifluoromethyl)phenyl]borate; **1** = for structure see above; **2** = (R)-Xyl-P-Phos; IPA = 2-propanol; THF = tetrahydrofuran; MeOH = methanol; LiOMe = lithium methanolate; DABCO = triethylenediamine.

**Example 10: Use of [(*R*)-Binap $RuCl_2$ (*R*)-DAIPEN] as catalyst**

14.50 g (31.80 mmol) of methyl 2-[3-[(*E*)-3-(2-(7-chloro-2-quinolinyl)ethenyl)phenyl]-3-oxopropyl]benzoate and 375 mg (1.64 mmol) triethylbenzylammonium chloride were placed in a 300 mL stainless steel autoclave equipped with a hollow shaft stirrer and a sampling tube under argon. 25 mL degassed 2-propanol and 36.2 mL (36.2 mmol) degassed, aqueous sodium hydroxide solution [1M] was successively added. 72.95 mg (0.066 mmol, S/C = 482:1) of [(*R*)-Binap $RuCl_2$ (*R*)-DAIPEN] was set under argon and dissolved in 120 mL of dry and degassed chlorobenzene. The catalyst solution was transferred into the autoclave under argon. Then, the autoclave was purged three times each with argon and hydrogen before the hydrogen pressure was set on a value of 5 bar at room temperature. The progress of the reaction was followed by taking samples after 3 and 6 hours. In addition, a hydrogen uptake curve was measured. After 8 hours no further hydrogen consumption was observed so that the pressure was released and the resulting heterogeneous mixture was divided into two equal portions. The two portions were subjected to two different work-up procedures resulting in a total of 13.37 g (92%) of the desired product methyl 2-[3-[(*E*)-3-(2-(7-chloro-2-quinolinyl)ethenyl)phenyl]-3(*S*)-3-hydroxypropyl]benzoate:

a) Non-aqueous work-up (filtration)

The first portion was directly transferred into a glass filter and filtered. The precipitate thus obtained was washed with 50 mL water, 50 mL toluene and 30 mL ethyl acetate/hexane (1:1). After drying, 3.96 g (27%) of methyl 2-[3-[(*E*)-3-(2-(7-chloro-2-quinolinyl)ethenyl)phenyl]-3(*S*)-3-hydroxypropyl]benzoate was obtained as tan, crystalline powder. The mother liquor and the washing solutions were applied to an aqueous work-up as outlined above. Thus, an additional amount of 2.11 g (15%) of methyl 2-[3-[(*E*)-3-(2-(7-chloro-2-quinolinyl)ethenyl)phenyl]-3(*S*)-3-hydroxypropyl]benzoate was obtained.

b) Aqueous work-up (extraction)

The second portion was treated with 600 mL ethyl acetate and 200 mL water. The layers were separated and the aqueous layer was extracted twice with 300 mL ethyl acetate. The combined organic layers were dried over sodium sulfate. Finally, the solvent was removed under reduced pressure yielding 7.30 g (50%) of methyl 2-[3-[(*E*)-3-(2-(7-chloro-2-quinolinyl)ethenyl)-phenyl]-3(*S*)-3-hydroxypropyl]benzoate as a resinous orange solid.

Purity: 98% by SFC (Chiralpak AD-H, 40% 2-propanol). Enantiomeric purity: 96.4% ee (*S*) by SFC (same conditions).

**Example 11: Use of [(*R*)-Binap $RuCl_2$ (*R*)-DAIPEN] as catalyst**

Chlorobenzene (891 g) was placed in a 2 L stainless steel autoclave equipped with a hollow shaft stirrer and a sampling tube under nitrogen. Under stirring, 226 g (0.495 mol) of freshly recrystallized (activated charcoal and Celite, THF/MeOH) methyl 2-[3-[(*E*)-3-(2-(7-chloro-2-quinolinyl)ethenyl)phenyl]-3-oxopropyl]benzoate was added. 5.6 g (0.025 mol; 5 mol%) triethylbenzylammonium chloride was dissolved in 131 g (2.185 mol) 2-propanol and the solution was transferred into the autoclave under nitrogen. Then, 198 mL (0.198 mol; 40 mol%) aqueous sodium hydroxide solution [1 M] was successively added. The autoclave was purged four times with nitrogen.

110 mg (0.099 mmol; S/C = 5000:1) [(*R*)-Binap $RuCl_2$ (*R*)-DAIPEN] was dissolved in 33 g (0.297 mol) of chlorobenzene. The catalyst solution was transferred into the autoclave under nitrogen, and the mixture was stirred. Then, the autoclave was purged at first four times with nitrogen and afterwards four times with hydrogen before the hydrogen pressure was set on a value of 10 bar at 23 °C.

During the first 30 to 120 minutes of the hydrogenation, no hydrogen consumption was observed due to activation of the catalyst. The progress of the reaction was followed by measuring the hydrogen uptake curve and taking samples at the end of the reaction to confirm completion. As soon as no further hydrogen consumption was observed (after about 15 hours), the hydrogen pressure was released and the resulting heterogeneous mixture purged four times with nitrogen.

100 g of chlorobenzene was added to the suspension. The slurry was stirred for 30 min at 5°C and finally transferred into a glass filter and filtered. The precipitate thus obtained was washed at 5 °C with 450 mL methanol/water (1:1, v/v) and finally with 226 mL methanol. After drying at 40 °C under reduced pressure, 204 g (86%) of methyl 2-[3-[(*E*)-3-(2-(7-chloro-2-quinolinyl)-ethenyl)phenyl]-3(S)-3-hydroxypropyl]benzoate monohydrate was obtained as yellowish, crystalline powder with a content of 99.7% (by $^1H$ NMR) and with an optical rotation of $[\alpha]_D^{25} = -26.4°$

## Claims

**1.** A process for the preparation of an optically active alcohol of formula

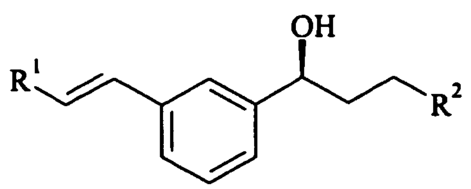

(I)

or its mirror image, wherein $R^1$ is unsubstituted or substituted heteroaryl and $R^2$ is phenyl or substituted aryl, by asymmetric hydrogenation of a ketone of formula

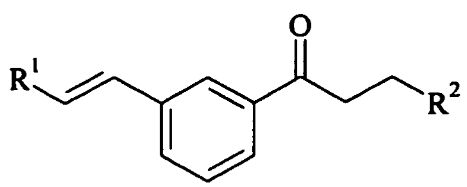

(II)

wherein $R^1$ and $R^2$ are as defined above,
**characterized in that** the asymmetric hydrogenation is conducted with hydrogen gas in the presence of a platinum metal complex catalyst comprising a chiral phosphine ligand,
wherein
the platinum metal is selected from the group consisting of ruthenium, rhodium and iridium; and the chiral phosphine ligand is of formula

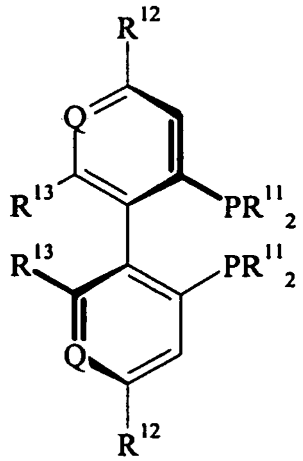

(III)

or its mirror image,
wherein
each $R^{11}$ is phenyl, 4-methylphenyl, 3,5-dimethylphenyl, furanyl or cyclohexyl;
each $R^{12}$ is hydrogen, $C_{1-4}$ alkyl or $C_{1-4}$ alkoxy;
and wherein

(a) each Q is nitrogen,
and each $R^{13}$ is $C_{1-4}$ alkyl or $C_{1-4}$ alkoxy; or
(b) each Q is $=CR^{14}-$, $R^{14}$ being selected from the group consisting of hydrogen, fluorine, chlorine, bromine, iodine and $C_{1-4}$ alkoxy;
and each $R^{13}$ is $C_{1-4}$ alkyl or $C_{1-4}$ alkoxy; or
(c) each Q is =CH-,
and both $R^{13}$ groups together form a moiety of formula $-O-(CH_2)_n-O-$, wherein n is an integer from 1 to 6; or
(d) each Q is $=CR^{15}-$, and each $R^{15}$ together with $R^{13}$ bound to the same benzene ring and together with said benzene ring form a ring system selected from the group consisting of naphthalene, tetralin, 2,3-dihydro-benzo[1,4]dioxine, unsubstituted or 2,2-dihalogen substituted benzo[1,3]dioxole, and *N*-methyl-2,3-dihydro-benzo[1,4]oxazine.

2. The process of claim 1, wherein the platinum metal complex catalyst further comprises a chiral diamine ligand.

3. The process of claim 2, wherein the chiral diamine ligand is of formula

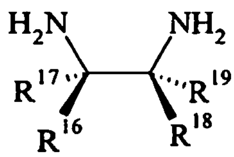

(IV)

wherein $R^{16}$ through $R^{19}$ is each independently hydrogen, cycloalkyl, linear or branched $C_{1-6}$ alkyl, or phenyl optionally substituted with one or more $C_{1-4}$ alkyl or $C_{1-4}$ alkoxy groups.

4. The process of claim 3, wherein $R^{16}$ is isopropyl; $R^{17}$ is hydrogen; and $R^{18}$ and $R^{19}$ are 4-methoxyphenyl.

**5.** The process of any of claims 1 to 4, wherein $R^1$ is a heterocyclic group of formula

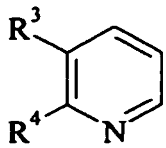

(V)

wherein $R^3$ and $R^4$ together form a moiety of formula $-S-CR^5=CR^6-$, with the proviso that the sulfur atom is directly bound to the carbon atom in position 3 of the pyridine moiety;
or alternatively $R^3$ and $R^4$ together form a moiety of formula $-CR^5=CR^6-CR^7=CR^8-$, with the proviso that the carbon atom attached to $R^5$ is directly bound to the carbon atom in position 3 of the pyridine moiety,
and each of $R^5$ through $R^8$ is independently hydrogen or halogen.

**6.** The process of any of claims 1 to 5, wherein $R^2$ is $-C_6H_4R^9$, wherein $R^9$ is selected from the group consisting of halogen, $C_{1-4}$ alkyl, branched or linear $C_{2-4}$ alkenyl, $C_{5-6}$ cycloalkyl, phenyl, $C_{1-4}$ alkoxy, $C_{1-4}$ alkylthio, carboxy, ($C_{1-4}$ alkoxy)carbonyl , ($C_{1-4}$ alkoxy sulfonyl, -T-O-$R^{10}$, wherein T is branched or linear $C_{1-8}$ alkanediyl and $R^{10}$ is selected from the group consisting of hydrogen, methyl, substituted methyl, substituted ethyl, phenyl, substituted phenyl, substituted benzyl, pyridinylmethyl, substituted pyridinylmethyl, substituted silyl, $C_{1-6}$ acyl, substituted $C_{1-6}$ acyl, ($C_{1-4}$ alkoxy)carbonyl, substituted ($C_{1-4}$ alkoxy)carbonyl and aryloxycarbonyl; and
$R^9$ may be located at any position of the phenyl ring.

**7.** The process of claim 5, wherein $R^3$ and $R^4$ together form a moiety of $-CR^5=CR^6-CR^7=CR^8-$; $R^5$, $R^6$ and $R^8$ are hydrogen; and $R^7$ is chlorine; and
wherein $R^2$ is $-C_6H_4R^9$, and $R^9$ is methoxycarbonyl and located at position 2 of the phenyl ring.

**8.** The process of claim 5, wherein $R^3$ and $R^4$ together form a moiety of $-CR^5=CR^6-CR^7=CR^8-$; $R^5$, $R^6$ and $R^8$ are hydrogen; and $R^7$ is chlorine; and
wherein $R^2$ is $-C_6H_4R^9$, and $R^9$ is -T-O-$R^{10}$ wherein T is $-C(CH_3)_2-$ and $R^{10}$ is hydrogen or substituted methyl selected from the group consisting of tetrahydropyranyl, methoxymethyl and ethoxymethyl; and $R^9$ is located at position 2 of the phenyl ring.

**9.** The process of any of claims 1 to 8, wherein each $R^{11}$ is phenyl; each $R^{12}$ is hydrogen;
each Q is $=CR^{15}-$, and wherein each $R^{15}$ together with $R^{13}$ bound to the same benzene ring and together with said benzene ring form a naphthalene ring system.

**10.** The process of any of claims 1 to 9, wherein the process is conducted in the presence of a base.

**11.** The process of any of claims 1 to 9, wherein the process is conducted in the presence of a Lewis acid.

**12.** The process of any of claims 1 to 11, wherein the process is conducted in the presence of a phase transfer catalyst.

**13.** The process of any of claims 1 to 12, wherein the process is conducted at a pressure between 1 and 100 bar.

**14.** The process of any of claims 1 to 13, wherein the process is conducted at an amount of the ketone of formula II (substrate) at a molar ratio relative to the catalyst (S/C) from 100:1 to 100,000:1.

**15.** The process of any of claims 1 to 14, wherein the process is conducted at a temperature between 0 °C and 100°C.

**16.** The process of any of claims 1 to 15 for synthesizing an optically active alcohol of formula I as an intermediate in the preparation of 1-[[[1-[(*E*)-3-(2-(7-chloro-2-quinolinyl)ethenyl)phenyl]-3(*R*)-[2-(1-hydroxy-1-methylethyl)phenyl] propyl]-thio]methyl]cyclopropaneacetic acid (montelukast) or montelukast sodium.

**Patentansprüche**

**1.** Ein Verfahren zur Herstellung eines optisch aktiven Alkohols der Formel

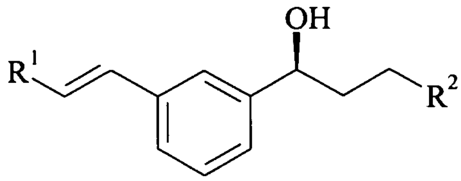

(I)

oder deren Spiegelbild, wobei $R^1$ für unsubstituiertes oder substituiertes Heteroaryl steht und $R^2$ für Phenyl oder substituiertes Aryl steht,
durch asymmetrische Hydrierung eines Ketons der Formel

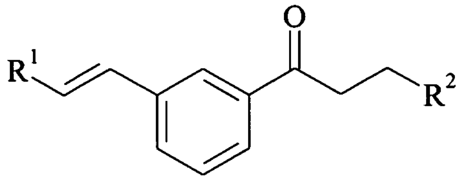

(II)

wobei $R^1$ und $R^2$ wie oben definiert sind,
**dadurch gekennzeichnet, dass** die asymmetrische Hydrierung mit Wasserstoffgas in Gegenwart eines Platinmetallkomplexkatalysators, der einen chiralen Phosphinliganden umfasst, durchgeführt wird, wobei das Platinmetall ausgewählt ist aus der Gruppe bestehend aus Ruthenium, Rhodium und Iridium; und der chirale Phosphinligand die Formel

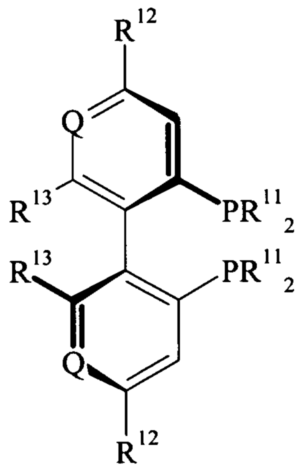

(III)

oder deren Spiegelbild hat, wobei
$R^{11}$ jeweils für Phenyl, 4-Methylphenyl, 3,5-Dimethylphenyl, Furanyl oder Cyclohexyl steht;
$R^{12}$ jeweils für Wasserstoff, $C_{1-4}$-Alkyl oder $C_{1-4}$-Alkoxy steht;
und wobei

(a) Q jeweils für Stickstoff steht,
und $R^{13}$ jeweils für $C_{1-4}$-Alkyl oder $C_{1-4}$-Alkoxy steht; oder
(b) Q jeweils für $=CR^{14}$- steht, wobei $R^{14}$ aus der Gruppe bestehend aus Wasserstoff, Fluor, Chlor, Brom, Iod und $C_{1-4}$-Alkoxy ausgewählt ist; und wobei $R^{13}$ jeweils für $C_{1-4}$-Alkyl oder $C_{1-4}$-Alkoxy steht; oder
(c) Q jeweils für =CH- steht,

und beide $R^{13}$-Gruppen zusammen eine Gruppierung der Formel -O-$(CH_2)_n$-O- bilden, wobei n für eine ganze Zahl von 1 bis 6 steht; oder (d) Q jeweils für $=CR^{15}$- steht und $R^{15}$ jeweils zusammen mit $R^{13}$an den gleichen Benzolring gebunden ist und zusammen mit diesem Benzolring ein Ringsystem bildet, das ausgewählt ist aus der Gruppe bestehend aus Naphthalin, Tetralin, 2,3-Dihydrobenzo[1,4]dioxin, unsubstituiertem oder 2,2-dihalogensubstituiertem Benzo[1,3]dioxol und *N*-methyl-2,3-dihydrobenzo[1,4]oxazin.

**2.** Das Verfahren nach Anspruch 1, wobei der Platinmetallkomplexkatalysator ferner einen chiralen Diaminliganden umfasst.

**3.** Das Verfahren nach Anspruch 2, wobei der chirale Diaminligand die Formel

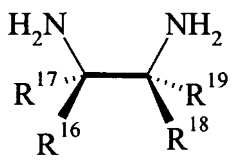

(IV)

hat, wobei $R^{16}$ bis $R^{19}$ jeweils unabhängig voneinander für Wasserstoff, Cycloalkyl, geradkettiges oder verzweigtes $C_{1-6}$-Alkyl oder Phenyl, das wahlweise durch eine oder mehrere $C_{1-4}$-Alkyl- oder $C_{1-4}$-Alkoxygruppen substituiert ist, steht.

**4.** Das Verfahren nach Anspruch 3, wobei $R^{16}$ für Isopropyl steht, $R^{17}$ für Wasserstoff steht, und $R^{18}$ und $R^{19}$ für 4-Methoxyphenyl stehen.

**5.** Das Verfahren nach einem der Ansprüche 1 bis 4, wobei $R^1$ für eine heterocyclische Gruppe der Formel

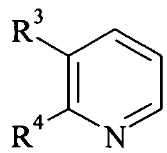

(V)

steht, wobei $R^3$ und $R^4$ zusammen eine Gruppierung der Formel $-S-CR^5=CR^6$-bilden, mit der Maßgabe, dass das Schwefelatom direkt an das Kohlenstoffatom in Position 3 der Pyridingruppierung gebunden ist;
oder alternativ $R^3$ und $R^4$ zusammen eine Gruppierung der Formel $-CR^5=CR^6-CR^7=CR^8$- bilden, mit der Maßgabe, dass das an $R^5$ gebundene Kohlenstoffatom direkt an das Kohlenstoffatom in Position 3 der Pyridingruppierung gebunden ist;
und $R^5$ bis $R^8$ jeweils unabhängig für Wasserstoff oder Halogen stehen.

**6.** Das Verfahren nach einem der Ansprüche 1 bis 5, wobei $R^2$ für $-C_6H_4R^9$ steht, wobei $R^9$ ausgewählt ist aus der Gruppe bestehend aus Halogen, $C_{1-4}$-Alkyl, verzweigtes oder geradkettiges $C_{2-4}$-Alkenyl, $C_{5-6}$-Cycloalkyl, Phenyl, $C_{1-4}$-Alkoxy, $C_{1-4}$-Alkylthio, Carboxy, ($C_{1-4}$-Alkoxy)carbonyl, ($C_{1-4}$-Alkoxy)-sulfonyl, $-T-O-R^{10}$, wobei T für verzweigtes oder geradkettiges $C_{1-8}$-Alkandiyl steht und $R^{10}$ ausgewählt ist aus der Gruppe bestehend aus Wasserstoff, Methyl, substituiertes Methyl, substituiertes Ethyl, Phenyl, substituiertes Phenyl, substituiertes Benzyl, Pyridinylmethyl, substituiertes Pyridinylmethyl, substituiertes Silyl, $C_{1-6}$-Acyl, substituiertes $C_{1-6}$-Acyl, ($C_{1-4}$-Alkoxy)carbonyl, substituiertes ($C_{1-4}$-Alkoxy)carbonyl und Aryloxycarbonyl; und
$R^9$ sich in jeder beliebigen Position des Phenylrings befinden kann.

**7.** Das Verfahren nach Anspruch 5, wobei $R^3$ und $R^4$ zusammen eine Gruppierung der Formel $-CR^5=CR^6-CR^7=CR^8$- bilden; $R^5$, $R^6$ und $R^8$ für Wasserstoff stehen; und $R^7$ für Chlor steht; und
wobei $R^2$ für $-C_6H_4R^9$ steht und $R^9$ für Methoxycarbonyl steht und sich in Position 2 des Phenylrings befindet.

**8.** Das Verfahren nach Anspruch 5, wobei $R^3$ und $R^4$ zusammen eine Gruppierung der Formel $-CR^5=CR^6-CR^7=CR^8$- bilden; $R^5$, $R^6$ und $R^8$ für Wasserstoff stehen; und $R^7$ für Chlor steht; und

wobei $R^2$ für $-C_6H_4R^9$ steht, und $R^9$ für $-T-O-R^{10}$ steht, wobei T für $-C(CH_3)_2$-steht und $R^{10}$ für Wasserstoff oder substituiertes Methyl ausgewählt aus der Gruppe bestehend aus Tetrahydropyranyl, Methoxymethyl und Ethoxymethyl steht; und $R^9$ sich in Position 2 des Phenylrings befindet.

**9.** Das Verfahren nach einem der Ansprüche 1 bis 8, wobei $R^{11}$ jeweils für Phenyl steht; $R^{12}$ jeweils für Wasserstoff steht; Q jeweils für $=CR_{15}$- steht, und wobei $R^{15}$ jeweils zusammen mit $R^{13}$ an den gleichen Benzolring gebunden ist und zusammen mit diesem Benzolring ein Naphthalinringsystem bildet.

**10.** Das Verfahren nach einem der Ansprüche 1 bis 9, wobei das Verfahren in Gegenwart einer Base durchgeführt wird.

**11.** Das Verfahren nach einem der Ansprüche 1 bis 9, wobei das Verfahren in Gegenwart einer Lewis-Säure durchgeführt wird.

**12.** Das Verfahren nach einem der Ansprüche 1 bis 11, wobei das Verfahren in Gegenwart eines Phasentransferkatalysators durchgeführt wird.

**13.** Das Verfahren nach einem der Ansprüche 1 bis 12, wobei das Verfahren bei einem Druck zwischen 1 und 100 bar durchgeführt wird.

**14.** Das Verfahren nach einem der Ansprüche 1 bis 13, wobei das Verfahren mit einer Menge des Ketons der Formel II (Substrat) in einem Molverhältnis, bezogen auf den Katalysator (S/C), von 100:1 bis 100.000:1 durchgeführt wird.

**15.** Das Verfahren nach einem der Ansprüche 1 bis 14, wobei das Verfahren bei einer Temperatur zwischen 0°C und 100°C durchgeführt wird.

**16.** Das Verfahren nach einem der Ansprüche 1 bis 15 zur Synthese eines optisch aktiven Alkohols der Formel I als Zwischenprodukt bei der Herstellung von 1-[[[1-[(*E*)-3-(2-(7-Chlor-2-chinolinyl)ethenyl)phenyl]-3(*R*)-[2-(1-hydroxy-1-methylethyl)phenyl]propyl]thio]methyl]cyclopropanessigsäure (Montelukast) oder Montelukast Natrium.

## Revendications

**1.** Procédé de préparation d'un alcool optiquement actif de formule

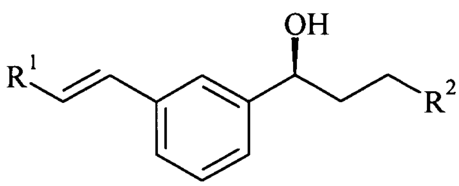

(I)

ou son image dans un miroir, dans laquelle $R^1$ est hétéroaryle substitué ou non substitué et $R^2$ est phényle ou aryle substitué,
par hydrogénation asymétrique d'une cétone de formule

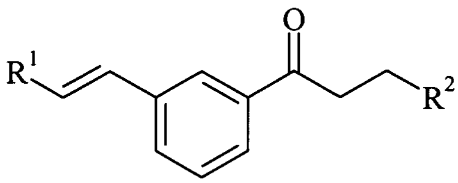

(II)

dans laquelle $R^1$ et $R^2$ sont tels que définis ci-dessus,
**caractérisé en ce que** l'hydrogénation asymétrique est effectuée avec de l'hydrogène gazeux en présence d'un catalyseur de complexe de platine métallique comprenant un ligand phosphine chiral,
dans lequel

le platine métallique est choisi dans le groupe constitué de ruthénium, de rhodium et d'iridium, et le ligand phosphine chiral est de formule

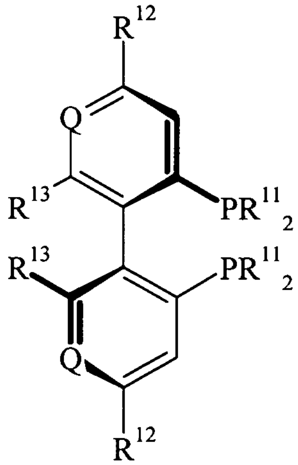

(III)

ou son image dans un miroir,
dans laquelle chaque $R^{11}$ est phényle, 4-méthylphényle, 3,5-diméthylphényle, furanyle ou cyclohexyle ;
chaque $R^{12}$ est hydrogène, alkyle en $C_{1-4}$ ou alcoxy en $C_{1-4}$ ;
et dans laquelle

(a) chaque Q est azote,
et chaque $R^{13}$ est alkyle en $C_{1-4}$ ou alcoxy en $C_{1-4}$ ; ou
(b) chaque Q est $=CR^{14}-$, $R^{14}$ étant choisi dans le groupe constitué d'hydrogène, de fluor, de chlore, de brome, d'iode et d'alcoxy en $C_{1-4}$; et chaque $R^{13}$ est alkyle en $C_{1-4}$ ou alcoxy en $C_{1-4}$ ; ou
(c) chaque Q est =CH- ;
et les deux groupements $R^{13}$ forment ensemble un motif de formule - $O-(CH_2)_n-O-$, dans laquelle *n* est un entier de 1 à 6 ; ou
(d) chaque Q est $=CR^{15}-$, et chaque $R^{15}$ ensemble avec $R^{13}$ lié au même cycle benzène et ensemble avec ledit cycle benzène forment un système de cycle choisi dans le groupe constitué de naphtalène, de tétraline, de 2,3-dihydro-benzo[1,4]dioxyne, de benzo[1,3]dioxole non substitué ou 2,2-dihalogène substitué, et de *N*-méthyl-2,3-dihydro-benzo[1,4]oxazine.

**2.** Procédé selon la revendication 1, **caractérisé en ce que** le catalyseur de complexe de platine métallique comprend en outre un ligand diamine chiral.

**3.** Procédé selon la revendication 2, **caractérisé en ce que** le ligand diamine chiral est de formule

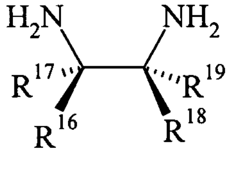

(IV)

dans laquelle $R^{16}$ à $R^{19}$ sont chacun indépendamment hydrogène, cycloalkyle, alkyle en $C_{1-6}$ linéaire ou ramifié, ou phényle éventuellement substitué par un ou plusieurs groupements alkyle en $C_{1-4}$ ou alcoxy en $C_{1-4}$.

**4.** Procédé selon la revendication 3, **caractérisé en ce que** $R^{16}$ est isopropyle ; $R^{17}$ est hydrogène ; et $R^{18}$ et $R^{19}$ sont 4-méthoxyphényle.

**5.** Procédé selon l'une quelconque des revendications 1 à 4, **caractérisé en ce que** $R^1$ est un groupement hétéro-

cyclique de formule

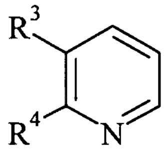

(V)

dans laquelle $R^3$ et $R^4$ forment ensemble un motif de formule $-S-CR^5=CR^6-$, à condition que l'atome de soufre soit lié directement à l'atome de carbone en position 3 du motif pyridine ;
ou selon une alternative $R^3$ et $R^4$ forment ensemble un motif de formule - $-CR^5=CR^6-CR^7=CR^8-$, à condition que l'atome de carbone lié à $R^5$ soit lié directement à l'atome de carbone en position 3 du motif pyridine ;
et chacun de $R^5$ à $R^8$ est indépendamment hydrogène ou halogène.

6. Procédé selon l'une quelconque des revendications 1 à 5, **caractérisé en ce que** $R^2$ est $-C_6H_4R^9$, où $R^9$ est choisi dans le groupe constitué d'halogène, d'alkyle en $C_{1-4}$, d'alcényle en $C_{2-4}$ ramifié ou linéaire, de cycloalkyle en $C_{5-6}$, de phényle, d'alcoxy en $C_{1-4}$, d'alkylthio en $C_{1-4}$, de carboxy, d'alcoxycarbonyle en $C_{1-4}$, d'alcoxysulfonyle en $C_{1-4}$, de $-T-O-R^{10}$, où T est alcanediyle en $C_{1-8}$ ramifié ou linéaire et $R^{10}$ est choisi dans le groupe constitué d'hydrogène, de méthyle, de méthyle substitué, d'éthyle substitué, de phényle, de phényle substitué, de benzyle substitué, de pyridinylméthyle, de pyridinylméthyle substitué, de silyle substitué, d'acyle en $C_{1-6}$, d'acyle en $C_{1-6}$ substitué, d'alcoxycarbonyle en $C_{1-4}$, d'alcoxycarbonyle en $C_{14}$ substitué et d'aryloxycarbonyle ; et
$R^9$ peut être situé dans n'importe quelle position du cycle phényle.

7. Procédé selon la revendication 5, **caractérisé en ce que** $R^3$ et $R^4$ forment ensemble un motif $-CR^5=CR^6-CR^7=CR^8-$; $R^5$, $R^6$ et $R^8$ sont hydrogène ; et $R^7$ est chlore ; et
où $R^2$ est $-C_6H_4R^9$, et $R^9$ est méthoxycarbonyle et est situé en position 2 du cycle phényle.

8. Procédé selon la revendication 5, **caractérisé en ce que** $R^3$ et $R^4$ forment ensemble un motif $-CR^5=CR^6-CR^7=CR^8-$; $R^5$, $R^6$ et $R^8$ sont hydrogène ; et $R^7$ est chlore ; et
où $R^2$ est $-C_6H_4R^9$, et $R^9$ est - $T-O-R^{10}$, où T est $-C(CH_3)_2-$ et $R^{10}$ est hydrogène ou méthyle substitué choisi dans le groupe constitué de tétrahydropyranyle, de méthoxyméthyle et d'éthoxyméthyle ; et $R^9$ est situé en position 2 du cycle phényle.

9. Procédé selon l'une quelconque des revendications 1 à 8, **caractérisé en ce que** chaque $R^{11}$ est phényle; chaque $R^{12}$ est hydrogène ; chaque Q est $=CR^{15}-$, et où chaque $R^{15}$ ensemble avec $R^{13}$ lié au même cycle benzène et ensemble avec ledit cycle benzène forment un système de cycle naphtalène.

10. Procédé selon l'une quelconque des revendications 1 à 9, **caractérisé en ce que** le procédé est effectué en présence d'une base.

11. Procédé selon l'une quelconque des revendications 1 à 9, **caractérisé en ce que** le procédé est effectué en présence d'un acide de Lewis.

12. Procédé selon l'une quelconque des revendications 1 à 11, **caractérisé en ce que** le procédé est effectué en présence d'un catalyseur de transfert de phase.

13. Procédé selon l'une quelconque des revendications 1 à 12, **caractérisé en ce que** le procédé est effectué à une pression comprise entre 1 et 100 bars.

14. Procédé selon l'une quelconque des revendications 1 à 13, **caractérisé en ce que** le procédé est effectué à une quantité de cétone de formule (II) (substrat) à un rapport molaire par rapport au catalyseur (S/C) de 100:1 à 100.000:1.

15. Procédé selon l'une quelconque des revendications 1 à 14, **caractérisé en ce que** le procédé est effectué à une température comprise entre 0°C et 100°C.

16. Procédé selon l'une quelconque des revendications 1 à 15, pour la synthèse d'un alcool optiquement actif de formule (I) en tant qu'intermédiaire dans la préparation de l'acide 1-[[[1-[(*E*)-3-(2-(7-chloro-2-quinoléinyl)éthényl)phényl]-3(*R*)-[2-(1-hydroxy-1-méthyléthyl)phényl]propyl]-thio]méthyl]cyclopropaneacétique (montélukast) ou montélukast sodique.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0480717 A
- US 20060223999 A
- WO 2006008562 A
- EP 0480717 B


### Non-patent literature cited in the description


- **D. Cai ; J. F. Payack ; D. R. Bender ; D. L. Hughes ; T. R. Verhoeven ; P. J. Reider.** *Org. Synth.,* 1998, vol. 76, 6-11